# EUROPEAN PATENT APPLICATION

(11) **EP 4 108 114 A1**
(43) Date of publication of application: **28.12.2022**
(21) Application number: 21180772.2
(22) Date of filing: 22.06.2021
(51) Int. Cl.: A41D 13/11, A61L 2/03, B01D 41/00, H01T 19/00

(54) **MEDICAL MASK RESTORING DEVICE**

(71) Applicant: Season Farm Technology Co., Ltd., Tainan City (TW)
(72) Inventor: Yang, Ching-Chieh, Kaohsiung City (TW)
(74) Representative: Cabinet Chaillot

(57) **Abstract**

A medical mask restoring device (1) includes an electric corona radiating net (2) producing an electric corona, an ion transmitter (12) connected with the electric corona radiating net, and at least one electricity supply (11) connected with the ion transmitter. Thus, the medical mask restoring device provides a restoring effect to the medical mask (A).

## Description

The present invention relates to a restoring (or recovering or renovating or rehabilitating) device and, more particularly, to a medical mask restoring device to keep a medical mask at a normal function or operation.

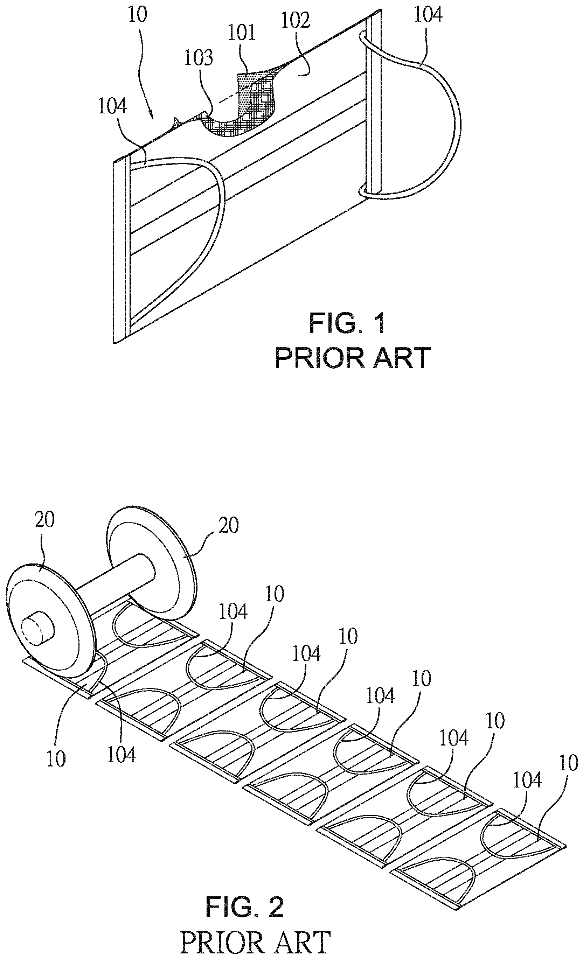

A conventional medical mask 10 in accordance with the prior art shown in FIGS. 1 and 2 comprises a waterproof (or spun-bond) non-woven fabric 101 served as an outer layer, a melt-blown non-woven fabric 103 served as an intermediate layer, and a composite fiber non-woven fabric 102 served as an inner layer. The conventional medical mask 10 has two ears 104 provided on two ends thereof. The waterproof non-woven fabric 101 is made of polypropylene (PP) to provide a waterproof effect. The composite fiber non-woven fabric 102 has a water absorbing function. The melt-blown non-woven fabric 103 contains a melt-blown fiber made of polypropylene (PP) that has a strong capacity to carry and store static charges. Thus, the melt-blown non-woven fabric 103 functions as a filtering layer to provide a filtering function so as to filter bacteria or virus by the static charges. The melt-blown non-woven fabric 103 contains static charges to capture and absorb the bacteria or virus by its static charges so as to achieve a sterilizing purpose. The melt-blown non-woven fabric 103 is charged by an electric corona to retain the static charges. In fabrication, rollers 20 are used to roll the conventional medical mask 10. The rollers 20 radiates an electric corona to charge the melt-blown non-woven fabric 103 so that the melt-blown non-woven fabric 103 contains the static charges. However, the static charges of the melt-blown non-woven fabric 103 decay gradually according to the environmental condition and the storage time. Especially, the melt-blown non-woven fabric 103 will lose the static charges under a condition of high temperature and high humidity. In addition, the static charges of the melt-blown non-woven fabric 103 are eliminated directly when approaching magnetic material. Further, the static charges of the melt-blown non-woven fabric 103 easily decompose when touching a person due to the charges contained in the human body.

The primary objective of the present invention is to provide a medical mask restoring device comprising an electric corona radiating net that produces an electric corona to keep or restore the function of a medical mask so as to provide a restoring effect to the medical mask.

In accordance with the present invention, there is provided a medical mask restoring device comprising an electric corona radiating net producing an electric corona, an ion transmitter connected with the electric corona radiating net, and at least one electricity supply connected with the ion transmitter.

Preferably, the medical mask restoring device further comprises a base, and an electrostatic charging face provided on a side of the base. The at least one electricity supply and the ion transmitter are mounted on the base. The electric corona radiating net is mounted on the electrostatic charging face.

Preferably, the medical mask restoring device further comprises a sensitive switch mounted on the electrostatic charging face. The electric corona radiating net is provided with a plurality of ion emitting portions. The ion transmitter is provided with a turn on/off portion which is connected with the sensitive switch.

Preferably, the medical mask restoring device further comprises a mounting seat, an electrostatic charging face provided on a side of the mounting seat, a clamping seat pivotally connected with the mounting seat, and a clamping face provided on the clamping seat. The electric corona radiating net is mounted on the electrostatic charging face and provided with a plurality of ion emitting portions. The at least one electricity supply and the ion transmitter are mounted on the clamping seat. The clamping face covers the electrostatic charging face when the clamping seat is pivoted to cover the mounting seat.

Preferably, the medical mask restoring device further comprises a cloth layer mounted on a front face of the electric corona radiating net, and a non-woven fabric layer mounted on a rear face of the electric corona radiating net. The electric corona radiating net is made of carbon fiber. The cloth layer, the electric corona radiating net, and the non-woven fabric layer construct a face mask which includes two ears provided on two sides thereof.

Preferably, the medical mask restoring device further comprises a cap for mounting the at least one electricity supply and the ion transmitter. The ion transmitter is provided with an emitting terminal. The electric corona radiating net is provided with a receiving terminal connected with the emitting terminal.

Preferably, the medical mask restoring device further comprises an elastic neck set for mounting the at least one electricity supply and the ion transmitter. The neck set has two distal ends. The ion transmitter is provided with an emitting terminal. The electric corona radiating net is provided with a receiving terminal connected with the emitting terminal.

Preferably, the medical mask restoring device further comprises a cloth layer mounted on a front face of the electric corona radiating net, the at least one electricity supply, and the ion transmitter, and a non-woven fabric layer mounted on a rear face of the electric corona radiating net, the at least one electricity supply, and the ion transmitter. The electric corona radiating net is made of carbon fiber. The cloth layer, the electric corona radiating net, the at least one electricity supply, the ion transmitter, and the non-woven fabric layer construct the medical mask restoring device which includes two ears provided on two sides thereof.

Further benefits and advantages of the present invention will become apparent after a careful reading of the detailed description with appropriate reference to the accompanying drawings.

In the drawings:
FIG. 1 is a perspective view of a conventional medical mask in accordance with the prior art.
FIG. 2 is a schematic view showing a roller for rolling the conventional medical mask as shown in FIG. 1.
FIG. 3 is a schematic view of a medical mask restoring device in accordance with the present invention.
FIG. 4 is a perspective view of a medical mask restoring device in accordance with the preferred embodiment of the present invention.
FIG. 5 is a perspective view showing usage of the medical mask restoring device as shown in FIG. 4.
FIG. 6 is a perspective view of a medical mask restoring device in accordance with another preferred embodiment of the present invention.
FIG. 7 is a perspective view showing usage of the medical mask restoring device as shown in FIG. 6.
FIG. 8 is a perspective view of a medical mask restoring device in accordance with a further preferred embodiment of the present invention.
FIG. 9 is a perspective view showing usage of the medical mask restoring device as shown in FIG. 8.
FIG. 10 is a perspective view showing usage of a medical mask restoring device in accordance with a further preferred embodiment of the present invention.
FIG. 11 is a perspective view showing usage of a medical mask restoring device in accordance with a further preferred embodiment of the present invention.

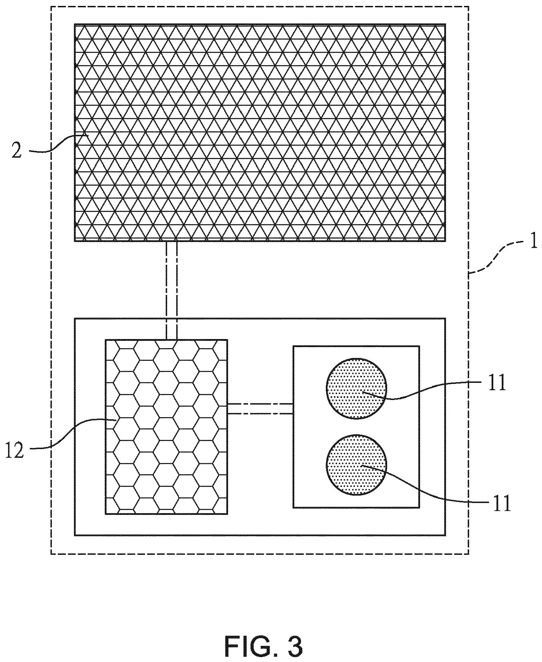

Referring to the drawings and initially to FIG. 3, a medical mask restoring device 1 in accordance with the preferred embodiment of the present invention comprises an electric corona radiating net 2 producing an electric corona, an ion transmitter 12 connected with the electric corona radiating net 2, and at least one electricity supply (or power supply) 11 connected with the ion transmitter 12. The at least one electricity supply 11 is a storage battery to provide little or micro electricity.

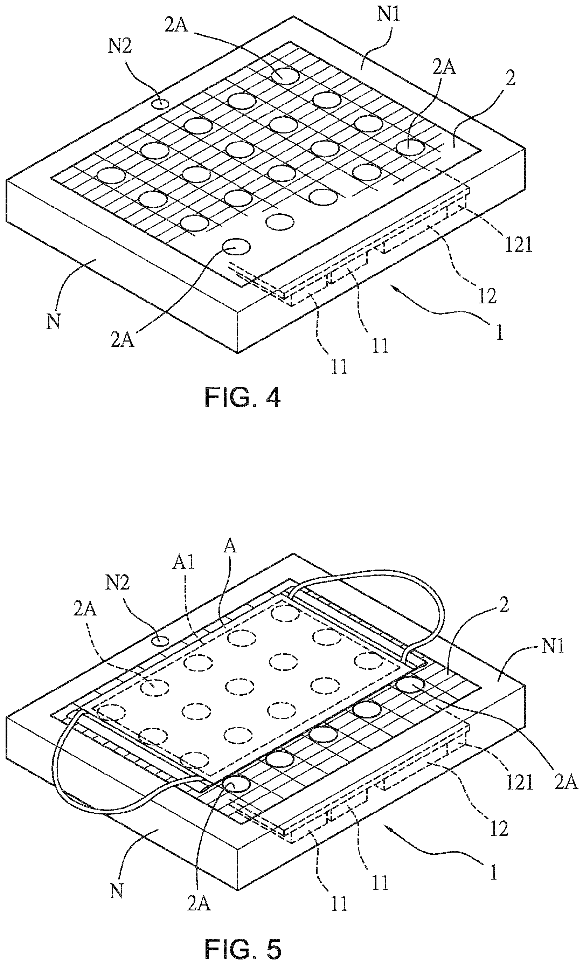

Referring to FIG. 4, the medical mask restoring device 1 further comprises a base N, an electrostatic charging face N1 provided on a side of the base N, and a sensitive switch (or sensor) N2 mounted on the electrostatic charging face N1. The at least one electricity supply 11 and the ion transmitter 12 are mounted on the base N. The electric corona radiating net 2 is mounted on the electrostatic charging face N1 and provided with a plurality of ion emitting portions 2A. The ion transmitter 12 is provided with a turn on/off portion 121 which is connected with the sensitive switch N2.

Referring to FIG. 5, when a medical mask A is placed on the electric corona radiating net 2, the sensitive switch N2 is activated to start the turn on/off portion 121 of the ion transmitter 12, such that the ion emitting portions 2A of the electric corona radiating net 2 are driven by the ion transmitter 12 to produce an electric corona. The electric corona radiating net 2 cooperates with the ion emitting portions 2A to produce an electric corona with a high efficiency for charging a melt-blown non-woven fabric A1 of the medical mask A. The melt-blown non-woven fabric A1 of the medical mask A captures and absorbs bacteria or virus by its static charges so as to achieve a sterilizing purpose. Thus, the melt-blown non-woven fabric A1 of the medical mask A is charged steadily and keeps the static charges constantly by the medical mask restoring device 1.

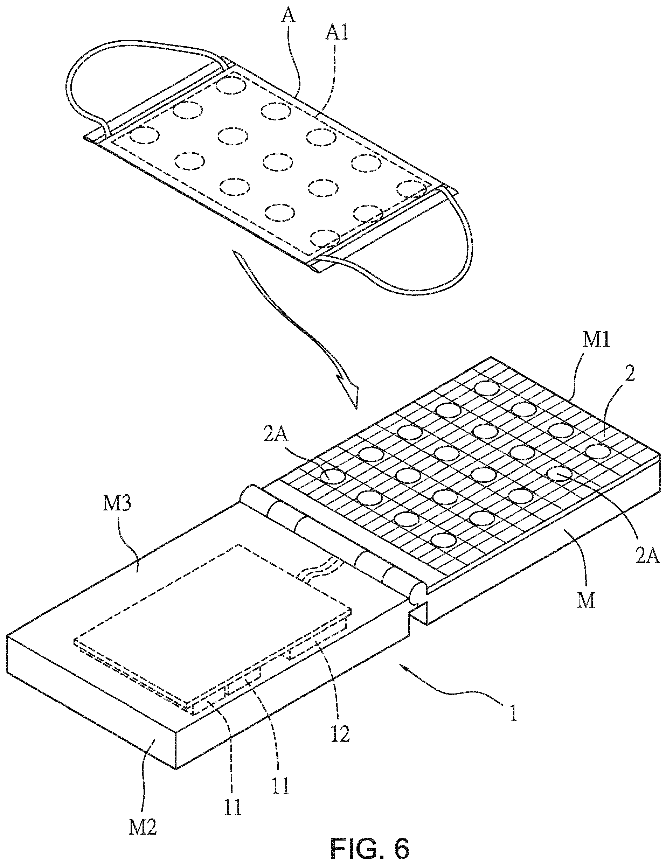

Referring to FIG. 6, the medical mask restoring device 1 further comprises a mounting seat M, an electrostatic charging face M1 provided on a side of the mounting seat M, a clamping seat M2 pivotally connected with the mounting seat M, and a clamping face M3 provided on the clamping seat M2. The electric corona radiating net 2 is mounted on the electrostatic charging face M1 and provided with a plurality of ion emitting portions 2A. The at least one electricity supply 11 and the ion transmitter 12 are mounted on the clamping seat M2. The clamping face M3 covers the electrostatic charging face M1 when the clamping seat M2 is pivoted to cover the mounting seat M.

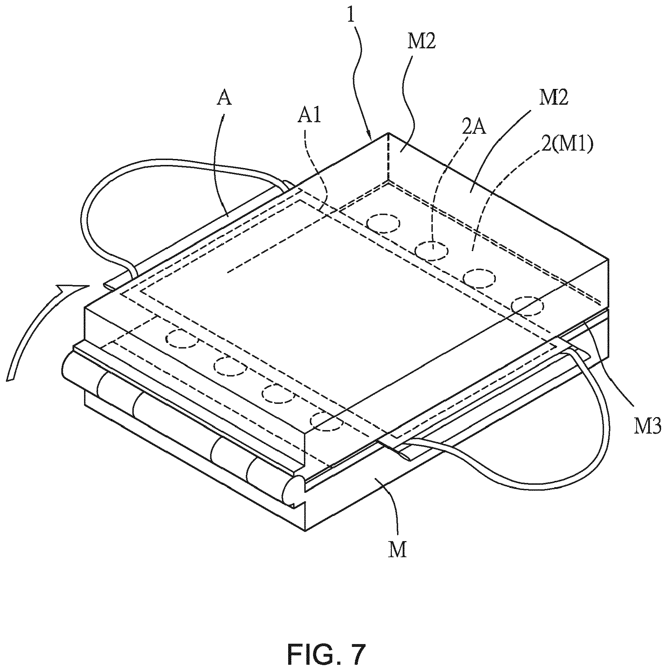

Referring to FIGS. 6 and 7, when the medical mask A is placed on the electrostatic charging face M1, the clamping seat M2 is pivoted to cover the mounting seat M, such that the medical mask A is sandwiched between the clamping face M3 and the electrostatic charging face M1. Thus, the at least one electricity supply 11 provides a weak electric power to start the ion transmitter 12, and the ion transmitter 12 also provides a weak electric power to the electric corona radiating net 2 so that the electric corona radiating net 2 cooperates with the ion emitting portions 2A to produce an electric corona for steadily charging the melt-blown non-woven fabric A1 of the medical mask A.

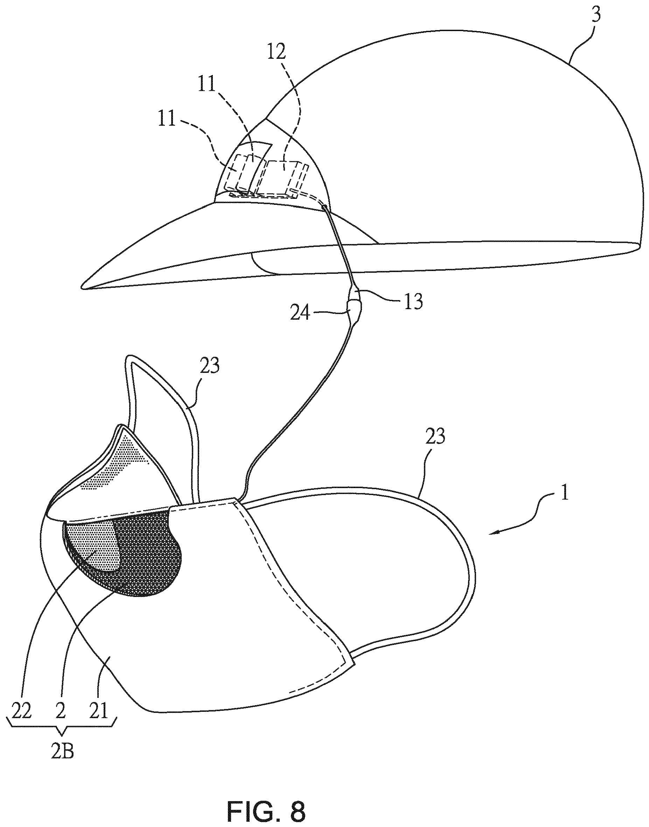

Referring to FIG. 8, the medical mask restoring device 1 further comprises a cloth layer 21 mounted on a front face of the electric corona radiating net 2, and a non-woven fabric layer 22 mounted on a rear face of the electric corona radiating net 2. The electric corona radiating net 2 is made of carbon fiber. The cloth layer 21, the electric corona radiating net 2, and the non-woven fabric layer 22 construct a face mask 2B which includes two ears 23 provided on two sides thereof. The medical mask restoring device 1 further comprises a cap 3 for mounting the at least one electricity supply 11 and the ion transmitter 12. The ion transmitter 12 is provided with an emitting terminal 13. The electric corona radiating net 2 is provided with a receiving terminal 24 connected with the emitting terminal 13.

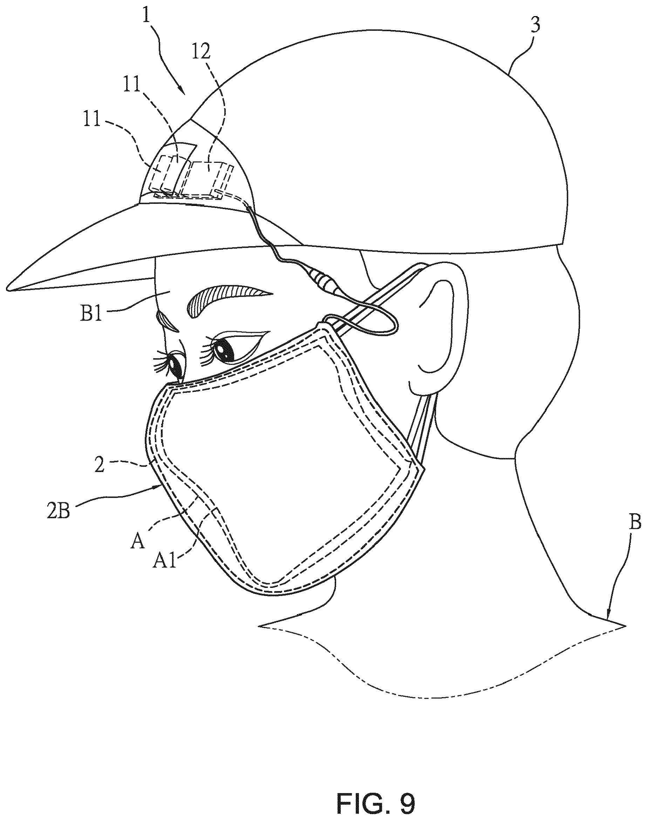

Referring to FIGS. 8 and 9, a user B wears the medical mask A and the face mask 2B simultaneously, with the face mask 2B being arranged outside the medical mask A. The cap 3 is put on the head B1 of the user B, with the emitting terminal 13 of the ion transmitter 12 connecting the receiving terminal 24 of the electric corona radiating net 2. Thus, the user B wears the medical mask A and the face mask 2B and puts on the cap 3 to carry the medical mask restoring device 1 conveniently. In operation, the at least one electricity supply 11 provides a weak electric power to start the ion transmitter 12, and the ion transmitter 12 also provides a weak electric power to the electric corona radiating net 2 so that the electric corona radiating net 2 produces an electric corona successively for steadily charging the melt-blown non-woven fabric A1 of the medical mask A. Thus, the melt-blown non-woven fabric A1 of the medical mask A is charged steadily and continuously and maintains the static charges constantly to capture and absorb bacteria or virus by its static charges so as to eliminate the bacteria or virus.

Alternatively, the cap 3 is undefined, and the at least one electricity supply 11 and the ion transmitter 12 are carried by the user B.

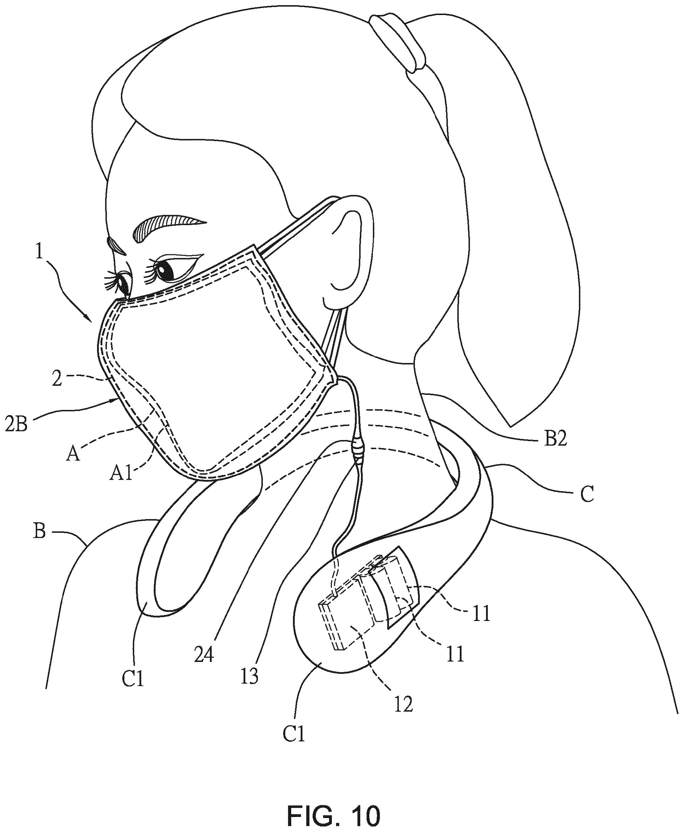

Referring to FIG. 10, the medical mask restoring device 1 further comprises an elastic neck set C for mounting the at least one electricity supply 11 and the ion transmitter 12. The neck set C has a C-shaped configuration and has two distal ends C1. The ion transmitter 12 is provided with the emitting terminal 13. The electric corona radiating net 2 is provided with the receiving terminal 24 connected with the emitting terminal 13. The neck set C is hung on the neck B2 of the user B, with the emitting terminal 13 of the ion transmitter 12 connecting the receiving terminal 24 of the electric corona radiating net 2. Thus, the user B wears the medical mask A and the face mask 2B and puts on the neck set C to carry the medical mask restoring device 1 conveniently.

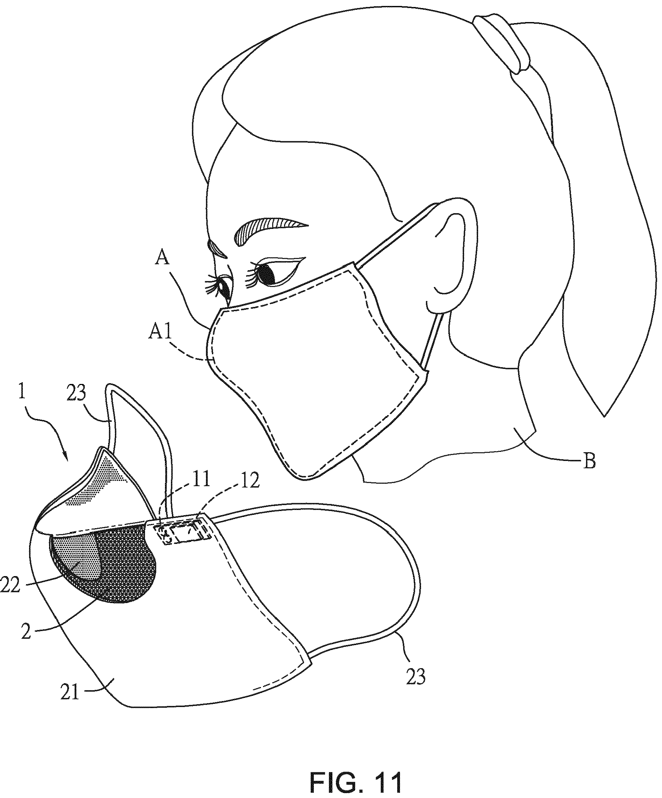

Referring to FIG. 11, the medical mask restoring device 1 further comprises a cloth layer 21 mounted on a front face of the electric corona radiating net 2, the at least one electricity supply 11, and the ion transmitter 12, and a non-woven fabric layer 22 mounted on a rear face of the electric corona radiating net 2, the at least one electricity supply 11, and the ion transmitter 12. The electric corona radiating net 2 is made of carbon fiber. The cloth layer 21, the electric corona radiating net 2, the at least one electricity supply 11, the ion transmitter 12, and the non-woven fabric layer 22 construct the medical mask restoring device 1 which includes two ears 23 provided on two sides thereof. The user B directly wears the medical mask A and the medical mask restoring device 1 to carry the medical mask restoring device 1 conveniently. The medical mask restoring device 1 cooperates with the medical mask A to directly charge the melt-blown non-woven fabric A1 so as to supplement the static charges of the melt-blown non-woven fabric A1.

Accordingly, when the medical mask A touches the medical mask restoring device 1, the at least one electricity supply 11 provides a weak electric power to start the ion transmitter 12 which also provides a weak electric power to start the electric corona radiating net 2 which produces an electric corona successively for steadily charging the melt-blown non-woven fabric A1 of the medical mask A, such that the melt-blown non-woven fabric A1 of the medical mask A is charged steadily and continuously and maintains the static charges constantly to capture and absorb bacteria or virus by its static charges so as to eliminate the bacteria or virus. In addition, the medical mask restoring device 1 provides a restoring effect to the medical mask A so that the melt-blown non-woven fabric A1 of the medical mask A preserves the static charges constantly without incurring decay, to enhance the sterilizing function and efficacy of the melt-blown non-woven fabric A1. Further, the electric corona radiating net 2 produces an electric corona successively to charge the melt-blown non-woven fabric A1 steadily, so that the melt-blown non-woven fabric A1 keeps the static charges constantly when being in use or restores the static charges when failing.

Regarding to one advantage of the present invention as shown in FIGS. 4 and 5, when the medical mask A is placed on the electrostatic charging face N1 of the medical mask restoring device 1, the at least one electricity supply 11 provides a weak electric power to start the ion transmitter 12 which also provides a weak electric power to start the electric corona radiating net 2 which produces an electric corona for charging the melt-blown non-woven fabric A1 of the medical mask A, such that the melt-blown non-woven fabric A1 holds the static charges constantly to capture and absorb bacteria or virus by its static effect so as to eliminate the bacteria or virus efficiently.

Regarding to another advantage of the present invention as shown in FIGS. 6 and 7, when the medical mask A is sandwiched between the clamping face M3 and the electrostatic charging face M1, the at least one electricity supply 11 provides a weak electric power to start the ion transmitter 12 which also provides a weak electric power to start the electric corona radiating net 2 which produces an electric corona for charging the melt-blown non-woven fabric A1 of the medical mask A, such that the melt-blown non-woven fabric A1 holds the static charges constantly to capture and absorb bacteria or virus by its static effect so as to eliminate the bacteria or virus efficiently.

Regarding to a further advantage of the present invention as shown in FIGS. 4 and 5, when the medical mask A approaches the electric corona radiating net 2 of the medical mask restoring device 1, the sensitive switch N2 is activated to start the ion transmitter 12, such that the ion emitting portions 2A and the electric corona radiating net 2 are driven by the ion transmitter 12 to produce an electric corona for charging the melt-blown non-woven fabric A1 of the medical mask A efficiently.

Regarding to a further advantage of the present invention as shown in FIGS. 8 and 9, when the user B wears the medical mask A and the face mask 2B and puts on the cap 3, the at least one electricity supply 11 provides a weak electric power to start the ion transmitter 12 which also provides a weak electric power to start the electric corona radiating net 2 which produces an electric corona for charging the melt-blown non-woven fabric A1 of the medical mask A, such that the melt-blown non-woven fabric A1 holds the static charges constantly to capture and absorb bacteria or virus by its static effect so as to eliminate the bacteria or virus efficiently.

Regarding to a further advantage of the present invention as shown in FIGS. 8 and 9, the cloth layer 21, the electric corona radiating net 2, and the non-woven fabric layer 22 construct the face mask 2B which includes two ears 23 provided on two sides thereof. Thus, the user B wears the medical mask A and the face mask 2B by the two ears 23 to carry the electric corona radiating net 2 of the medical mask restoring device 1 conveniently.

Regarding to a further advantage of the present invention as shown in FIGS. 8 and 9, the at least one electricity supply 11 and the ion transmitter 12 are mounted on the cap 3. Thus, the user B puts on the cap 3 to carry the at least one electricity supply 11 and the ion transmitter 12 of the medical mask restoring device 1 conveniently.

Regarding to a further advantage of the present invention as shown in FIG. 10, the at least one electricity supply 11 and the ion transmitter 12 are mounted on the neck set C. Thus, the user B puts on the neck set C to carry the at least one electricity supply 11 and the ion transmitter 12 of the medical mask restoring device 1 conveniently.

Regarding to a further advantage of the present invention as shown in FIG. 11, the cloth layer 21, the electric corona radiating net 2, the at least one electricity supply 11, the ion transmitter 12, and the non-woven fabric layer 22 construct the medical mask restoring device 1. Thus, the user B directly wears the medical mask A and the medical mask restoring device 1 to carry the medical mask restoring device 1 conveniently.

Although the invention has been explained in relation to its preferred embodiment(s) as mentioned above, it is to be understood that many other possible modifications and variations can be made without departing from the scope of the present invention. It is, therefore, contemplated that the appended claim or claims will cover such modifications and variations that fall within the scope of the invention.

## Claims

1. A medical mask restoring device (1) comprising:
an electric corona radiating net (2) producing an electric corona;
an ion transmitter (12) connected with the electric corona radiating net; and
at least one electricity supply (11) connected with the ion transmitter.

2. The medical mask restoring device as claimed in claim 1, further comprising:
a base (N); and
an electrostatic charging face (N1) provided on a side of the base;
wherein:
the at least one electricity supply and the ion transmitter are mounted on the base; and
the electric corona radiating net is mounted on the electrostatic charging face.

3. The medical mask restoring device as claimed in claim 2, further comprising:
a sensitive switch (N2) mounted on the electrostatic charging face;
wherein:
the electric corona radiating net is provided with a plurality of ion emitting portions (2A); and
the ion transmitter is provided with a turn on/off portion (121) which is connected with the sensitive switch.

4. The medical mask restoring device as claimed in claim 1, further comprising:
a mounting seat (M);
an electrostatic charging face (M1) provided on a side of the mounting seat;
a clamping seat (M2) pivotally connected with the mounting seat; and
a clamping face (M3) provided on the clamping seat;
wherein:
the electric corona radiating net is mounted on the electrostatic charging face and provided with a plurality of ion emitting portions (2A);
the at least one electricity supply and the ion transmitter are mounted on the clamping seat; and
the clamping face covers the electrostatic charging face when the clamping seat is pivoted to cover the mounting seat.

5. The medical mask restoring device as claimed in claim 1, further comprising:
a cloth layer (21) mounted on a front face of the electric corona radiating net; and
a non-woven fabric layer (22) mounted on a rear face of the electric corona radiating net;
wherein:
the electric corona radiating net is made of carbon fiber; and
the cloth layer, the electric corona radiating net, and the non-woven fabric layer construct a face mask (2B) which includes two ears (23) provided on two sides thereof.

6. The medical mask restoring device as claimed in claim 5, further comprising:
a cap (3) for mounting the at least one electricity supply and the ion transmitter;
wherein:
the ion transmitter is provided with an emitting terminal (13); and
the electric corona radiating net is provided with a receiving terminal (24) connected with the emitting terminal.

7. The medical mask restoring device as claimed in claim 5, further comprising:
an elastic neck set (C) for mounting the at least one electricity supply and the ion transmitter;
wherein:
the neck set has two distal ends (C1);
the ion transmitter is provided with an emitting terminal (13); and
the electric corona radiating net is provided with a receiving terminal (24) connected with the emitting terminal.

8. The medical mask restoring device as claimed in claim 1, further comprising:
a cloth layer (21) mounted on a front face of the electric corona radiating net, the at least one electricity supply, and the ion transmitter; and
a non-woven fabric layer (22) mounted on a rear face of the electric corona radiating net, the at least one electricity supply, and the ion transmitter;
wherein:
the electric corona radiating net is made of carbon fiber;
the cloth layer, the electric corona radiating net, the at least one electricity supply, the ion transmitter, and the non-woven fabric layer construct the medical mask restoring device which includes two ears (23) provided on two sides thereof.
